# EUROPEAN PATENT APPLICATION

(11) **EP 2 644 606 A1**
(43) Date of publication of application: **02.10.2013**
(21) Application number: 11843292.1
(22) Date of filing: 14.11.2011
(51) Int. Cl.: C07D 333/16, C09K 11/06, C07F 5/00

(54) **RARE EARTH METAL COMPLEX**

(30) Priority: 22.11.2010 JP 2010260326
(71) Applicant: Hitachi Chemical Co., Ltd., Chiyoda-ku Tokyo 100-6606 (JP)
(72) Inventor: YAMASHITA, Takeshi, Tsukuba-shi Ibaraki 300-4247 (JP)
(74) Representative: Albrecht, Thomas
(86) International application number: PCT/JP2011/076198
(87) International publication number: WO 2012/070420

(57) **Abstract**

Provided is a rare earth metal complex including a rare earth metal atom and a β-diketone compound coordinated to the rare earth metal atom, the β-diketone compound being represented by the following Formula (1). In Formula (1), R¹ represents a hydrogen atom, a halogen atom, an alkyl group, a perfluoroalkyl group, an alkoxy group, a perfuoroalkoxy group, a nitro group, an amino group, a sulfonyl group, a cyano group, a silyl group, a phosphone group, a diazo group, a mercapto group, an aryl group, an aralkyl group, an aryloxy group, an aryloxycarbonyl group, an allyl group, an acyl group, or an acyloxy group.

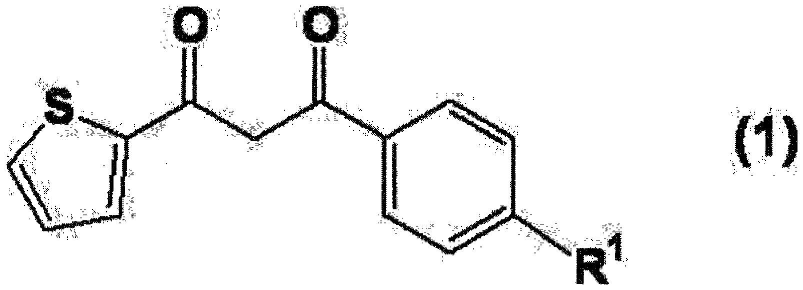

## Description

### [Technical Field]

The present invention relates to a rare earth metal complex that can be excited by excitation light having a longer wavelength than in conventional rare earth metal complexes.

### [Background Art]

Conventionally, various rare earth-based light emitting materials are known. In lighting apparatuses and display apparatuses, light emitting devices are used in which light of a discharge lamp or a semiconductor light emitting element is color-converted with a fluorescent material.

In recent years, particularly, fluorescent materials using a rare earth metal complex has been expected to be applied in a variety of fields in terms of having high solubility in solvents and high dispersibility in resin, unlike inorganic fluorescent materials. For example, there have been proposed various applications of fluorescent materials, such as fluorescent probes, bioimaging, ink for printing, sensors, wavelength conversion resin sheet, and lightning.

As a light emitting mechanism of a rare earth metal complex, there is known a mechanism in which a ligand absorbs light and the excitation energy thereof is transferred to a rare earth metal ion as a light emission center to excite the ion, thereby emitting light.
From the viewpoint of the application range of fluorescent materials, extension of excitation wavelength has been desired. However, changing the skeleton of the ligand to extend the excitation wavelength has sometimes reduced energy transfer efficiency between the ligand and the metal and therefore practically sufficient light emission intensity has not been obtainable.

In relation to the above circumstances, Japanese Patent Application Laid-Open (JP-A) No. 2005-252250 has proposed a rare earth metal complex that can be excited by a longer wavelength than in conventional rare earth metal complexes by sufficiently reducing impurities, crystal defects, and deactivation due to energy trapping in the process of energy transfer from ligand.

In addition, JP-A-2009-46577 has proposed a rare earth metal complex that can be excited at a longer wavelength than in conventional rare earth metal complexes by reacting a rare earth metal complex coordinated by phosphine oxide with a siloxane bond-containing compound to activate the f-f transition of a rare earth metal.

### SUMMARY OF INVENTION

### [Technical Problem]

However, in the rare earth metal complex described in JP-A-2005-252250, light emission intensity has sometimes been insufficient. Additionally, in some cases, it has been hard to say that the rare earth metal complex described in JP-A-2009-46577 has high general versatility, in terms of requiring hydro silicone as an essential ingredient.
In view of the problems, it is an object of the present invention to provide a rare earth metal complex that can be excited by excitation light having a longer wavelength than in the conventional rare earth metal complexes.

### [Solution to Problem]

Specific means for solving the problems are as follows.
<1> A rare earth metal complex including a rare earth metal atom and a β-diketone compound coordinated to the rare earth metal atom, the β-diketone compound being represented by the following Formula (1).

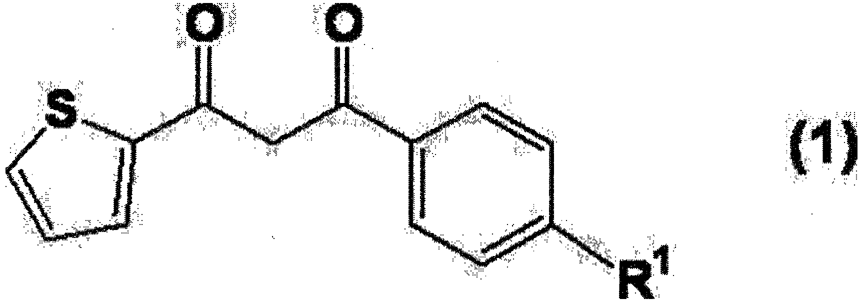

In Formula (1), R¹ represents a hydrogen atom, a halogen atom, an alkyl group, a perfluoroalkyl group, an alkoxy group, a perfluoroalkoxy group, a nitro group, an amino group, a sulfonyl group, a cyano group, a silyl group, a phosphone group, a diazo group, a mercapto group, an aryl group, an aralkyl group, an aryloxy group, an aryloxycarbonyl group, an allyl group, an acyl group or an acyloxy group.

<2> The rare earth metal complex according to the <1>, having a maximum absorption at a wavelength of 350 nm or more.

<3> The rare earth metal complex according to the <1> or <2>, represented by the following Formula (2).

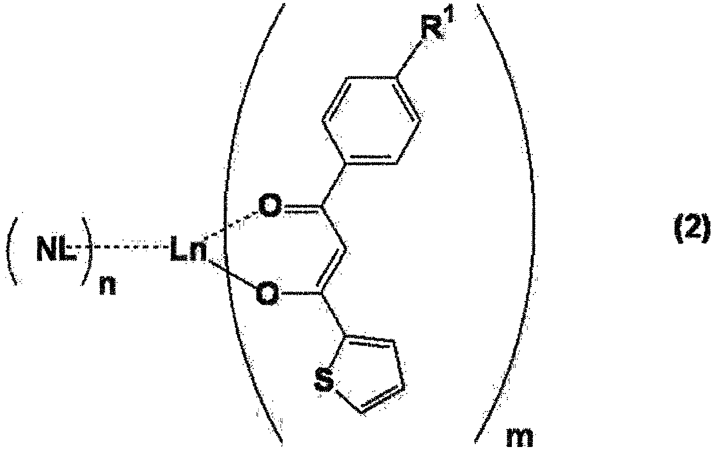

In Formula (2), Ln represents a rare earth metal atom; NL represents a neutral ligand; R¹ represents a hydrogen atom, a halogen atom, an alkyl group, a perfluoroalkyl group, an alkoxy group, a perfluoroalkoxy group, a nitro group, an amino group, a sulfonyl group, a cyano group, a silyl group, a phosphone group, a diazo group, a mercapto group, an aryl group, an aralkyl group, an aryloxy group, an aryloxycarbonyl group, an allyl group, an acyl group or an acyloxy group; n represents an integer from 1 to 5; and m represents an integer equal to a valence of Ln.

<4> The rare earth metal complex according to any one of the <1> to <3>, in which the rare earth metal atom is europium (Eu), terbium (Tb), erbium (Er), ytterbium (Yb), neodymium (Nd) or samarium (Sm).

<5> The rare earth metal complex according to any one of the <1> to <4>, in which R¹ in Formula (1) represents an electron attracting group.

<6> The rare earth metal complex according to any one of the <1> to <4>, in which R¹ in Formula (1) represents a halogen atom or a perfluoroalkyl group.

### [Advantageous Effects of Invention]

According to the present invention, there can be obtained a rare earth metal complex that can be excited by excitation light having a longer wavelength than in the conventional rare earth metal complexes.

### BRIEF DESCRIPTION OF DRAWINGS

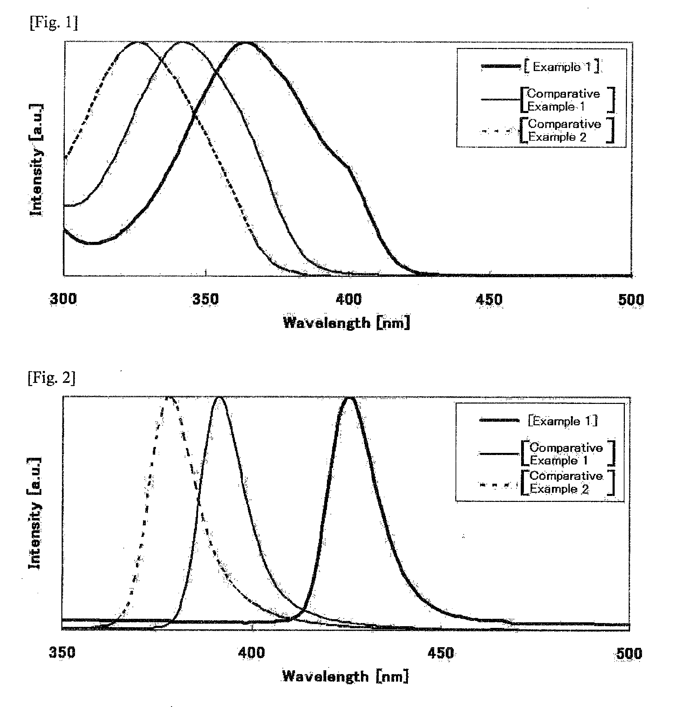

FIG. 1 illustrates the maximum absorption spectra of rare earth mental complexes obtained in Example 1 and Comparative Examples 1 and 2.
FIG. 2 illustrates the excitation spectra of the rare earth metal complexes obtained in Example 1 and Comparative Examples 1 and 2 in a solution.

### DESCRIPTION OF EMBODIMENTS

Herein, a numerical range described using the term "to" means a range including numerical values before and after "to" as a minimum value and a maximum value, respectively.

Regarding rare earth metal complexes including a β-diketone compound as a ligand, particularly when the central metal of the metal complexes has been Eu³⁺, many of the metal complexes have had a maximum absorption at a wavelength of 350 nm or less. Considering the form of utilization, it is desirable to shift toward longer excitation wavelengths of the rare earth metal complexes.

Herein, light emission of a rare earth metal complex occurs through energy transfer from a ligand. In order to cause the metal complex to emit light, in a relative relationship between energy levels of the ligand and the central metal, an excitation level of the ligand needs to be higher than an excitation level of the central metal. Accordingly, shifting toward longer excitation wavelength leads to restriction on the range of choice of the possible energy transfer, which is difficult in principle.

However, as a result of intensive and extensive investigation, the present inventor has found that when a β-diketone compound having a specific structure as a ligand has been coordinated to a rare earth metal, there can be obtained a rare earth metal complex that can be excited by excitation light having a longer wavelength than in the conventional rare earth metal complexes.

A rare earth metal complex according to the present invention includes a rare earth metal atom and a β-diketone compound coordinated to the rare earth metal atom, the β-diketone compound being represented by the following Formula (1):

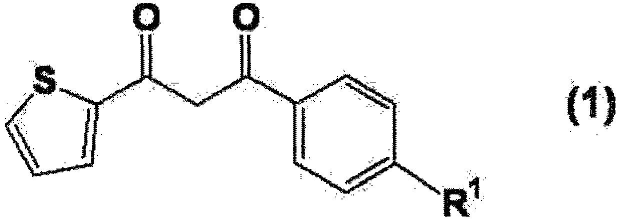

In Formula (1) above, R¹ represents a hydrogen atom, a halogen atom, an alkyl group, a perfluoroalkyl group, an alkoxy group, a perfluoroalkoxy group, a nitro group, an amino group, a sulfonyl group, a cyano group, a silyl group, a phosphone group, a diazo group, a mercapto group, an aryl group, an aralkyl group, an aryloxy group, an aryloxycarbonyl group, an allyl group, an acyl group or an acyloxy group.

Preferably, R¹ represents an electron attracting group from the viewpoint of structural stabilization. Specifically, R¹ represents preferably a halogen atom, a perfluoroalkyl group, a perfluoroalkoxy group, a nitro group, a sulfonyl group, a cyano group, a phosphone group, or a diazo group, preferably a halogen atom or a perfluoroalkyl group, more preferably a halogen atom or a perfluoroalkyl group having 1 to 3 carbon atoms, and still more preferably a halogen atom or a perfluoroalkyl group having 1 to 2 carbon atoms.

Specifically, R¹ represents preferably a fluorine atom, a chlorine atom, a bromine atom, an iodine atom, a trifluoromethyl group, a pentafluoroethyl group or a heptafluoropropyl group; more preferably a fluorine atom, a chlorine atom, a trifluoromethyl group or a pentafluoropropyl group; and still more preferably a hydrogen atom, a fluorine atom or a trifluoromethyl group.

The followings are specific examples of the β-diketone compound represented by Formula (1). However, the present invention is not limited thereto.

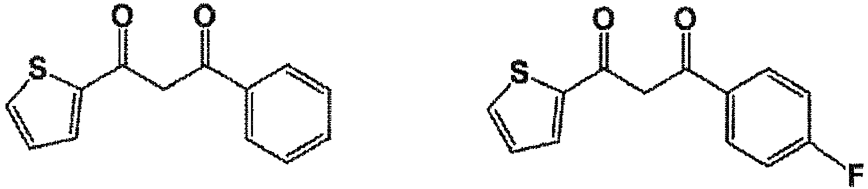

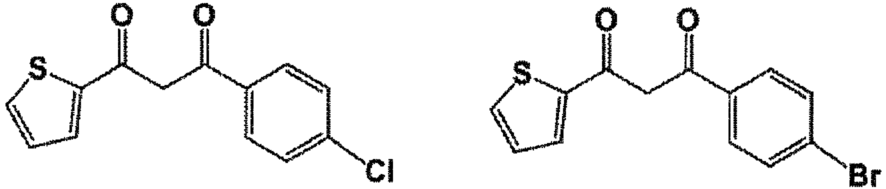

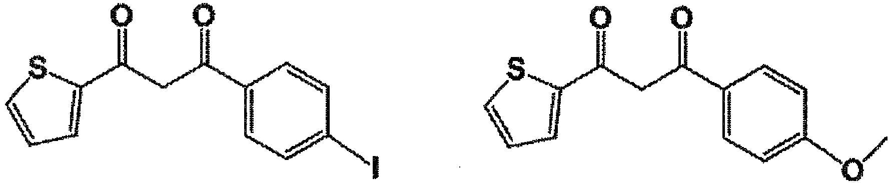

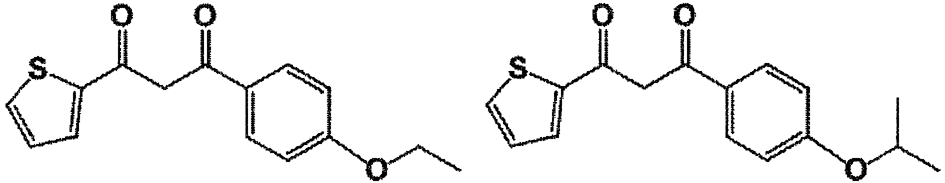

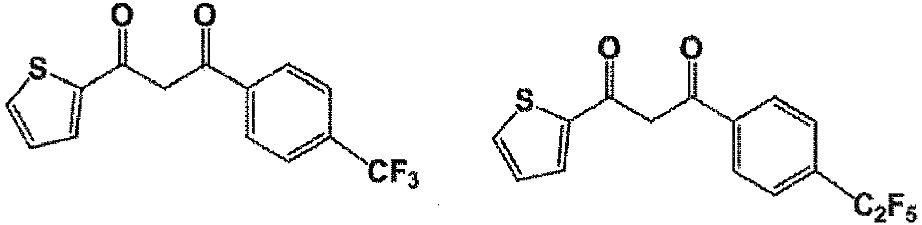

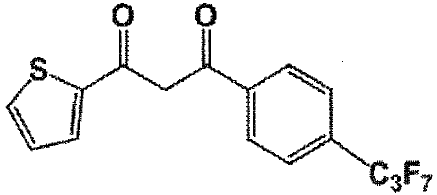

The β-diketone compound represented by Formula (1) can be obtained, for example, as indicated by the following reaction formula, by condensing 2-acetylthiophene with benzoate (for example, methyl benzoate) or 4-substituted benzoate (for example, methyl 4-fluorobenzoate) in the presence of a base. In the following formula, R² represents an alkyl group (preferably, an alkyl group having 1 to 4 carbon atoms), an aryl group or the like.

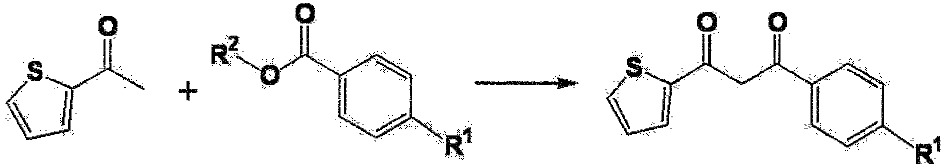

The rare earth metal atom included in the rare earth metal complex of the present invention is, from the viewpoint of the wavelength of light emission, preferably europium (Eu), terbium (Tb), erbium (Er), ytterbium (Yb), neodymium (Nd) or samarium (Sm); more preferably Eu, Sm or Tb; and particularly preferably Eu.

The rare earth metal complex including a β-diketone compound as a ligand according to the present invention is not limited as long as a total number of coordination to the rare earth metal atom is from 6 to 9. Examples of such complexes include a complex in which three molecules of β-diketonate as an anion with a valence of -1 are coordinated to a rare earth metal ion with a valence of +3, and a complex in which a Lewis basic neutral ligand is coordinated, as an auxiliary ligand, to the above-described complex, or a complex including four coordinated β-diketonate molecules and a cationic molecule for neutralizing a total valence.
Particularly, considering dispersibility in a medium and fluorescent properties as the fluorescent material, preferred is a complex including the three molecules of a β-diketonate compound coordinated to a rare earth metal and a neutral ligand as a Lewis basic.

The rare earth metal complex of the present invention is, from the viewpoint of the wavelength of light emission, preferably a complex represented by the following Formula (2):

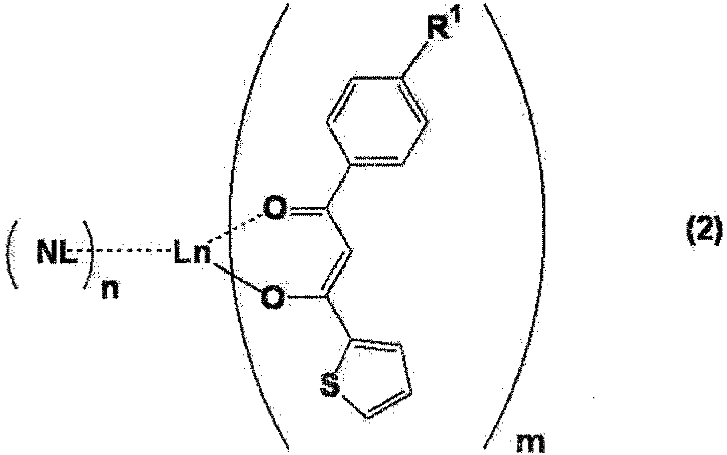

In Formula (2), Ln represents a rare earth metal atom; NL represents a neutral ligand; R¹ represents a hydrogen atom, a halogen atom, an alkyl group, a perfluoroalkyl group, an alkoxy group, a perfluoroalkoxy group, a nitro group, an amino group, a sulfonyl group, a cyano group, a silyl group, a phosphone group, a diazo group, a mercapto group, an aryl group, an aralkyl group, an aryloxy group, an aryloxycarbonyl group, an allyl group, an acyl group or an acyloxy group; n represents an integer from 1 to 5; and m represents an integer equal to a valence of Ln.

In Formula (2), examples of the rare earth metal atom represented by Ln include the rare earth metal atoms mentioned above, and suitable rare earth metal atoms are also the same as those above, so that explanations thereof here are omitted.
The R¹ in Formula (2) has the same definition as the R¹ in Formula (1) and the preferable range thereof is also the same as the range of the R¹ in Formula (1), so that an explanation thereof here is omitted.

The neutral ligand represented by NL, is not particularly limited as long as the ligand can be coordinated to the rare earth metal atom Ln. Examples of the neutral ligand include compounds including a nitrogen atom, an oxygen atom or a sulfur atom. Specific examples thereof include amins, amine oxides, phosphine oxides, ketones, sulfoxides, ethers and the like. These compounds may be selected alone or in combination.
In addition, when Ln represents Eu³⁺, the neutral ligand is selected such that the total coordination number of the Eu³⁺ is 7, 8 or 9.

Examples of the amines represented by the neutral ligand NL include pyridine, pyradine, quinoline, isoquinoline, 2,2'-bipyridine, 1,10-phenanthroline, each of which may have a substituent.
Examples of the amine oxides represented by the neutral ligand NL include N-oxides of the amines, such as pyridine-N-oxide, isoquinoline-N-oxide, 2,2'-bipyridine-N,N'-dioxide, and 1,10-phenanthroline-N,N'-dioxide, each of which may have a substituent.

Examples of the phosphine oxides represented by the neutral ligand include alkylphosphine oxides such as triphenylphosphine oxide, triethylphosphine oxide, and trioctylphosphine oxide, 1,2-ethylenebis (diphenylenephosphine oxide), (diphenylphosphineimide) triphenylphosphorane, triphenyl phosphate, and the like, each of which may have a substituent.
Examples of the ketones represented by the neutral ligand NL include dipyridylketone, benzophenone, and the like, each of which may have a substitutent.

Examples of the sulfoxides represented by the neutral ligand NL include diphenyl sulfoxide, dibenzyl sulfoxide, dioctyl sulfoxide, each of which may have a substitutent.
Examples of the ethers represented by the neutral ligand NL include ethylene glycol dimethyl ether and ethylene glycol dimethyl ether, each of which may have a substitutent.

In Formula (2), n represents an integer from 1 to 5, preferably an integer from 1 to 3, and more preferably an integer from 1 to 2.
In Formula (2), m represents an integer equal to a valence of Ln. For example, when Ln represents Eu³⁺, m representes 3.

In Formula (2), when the rare earth metal atom Ln represents Eu, the neutral ligand NL represents preferably an amine, a phosphine oxide or a sulfoxide; more preferably an amine or a phosphine oxide; and still more preferaly an amine. In addition, among amines, preferred is a neutral ligand NL represented by the following Formula (3):

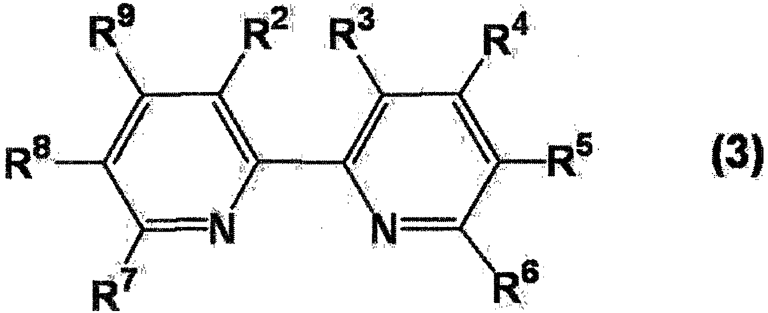

In Formula (3), R² to R⁹ each independentaly represent a hydrogen atom, an alkyl group or an aryl group. In addition, R² and R³, R³ and R⁴, R⁴ and R⁵, R⁵ and R⁶, R⁷ and R⁸, R⁸ and R⁹, and R⁹ and R², respectively, may bond to each other to form a ring.

Preferably, the neutral ligand is a bipyridine compound in which R² and R³ in Formula (3) each independently represent a hydrogen atom, or the neutral ligand is a phenathroline compound in which R² and R³ bond to each other to form a benzene ring.

R² to R⁹ in Formula (3) each independently preferably represent a hydrogen atom, an alkyl group having 1 to 9 carbon atoms or a phenyl group; more preferably represent a hydrogen atom, a methyl group, an ethyl group or a phenyl group; and still more preferably a hydrogen atom, a methyl group or a phenyl group.

When any of R⁴ to R⁹ in Formula (3) represents an alkyl group or an aryl group, preferably at least R⁵ or R⁸ (namely, the 5-position) represents an alkyl group or an aryl group.

Supecific examples of the neutral ligand represented by Formula (3) include, preferably, 2,2'-bipyridine, 1,10-phenanthroline, basophenanthroline, neocuproine, basocuproine, 5,5'-dimethyl-2,2'-bipyridine, 4,4'-dimethyl-2,2'-bipyridine, 6,6'-dimethyl-2,2'-bipyridine, 5-phenyl-2,2'-bipyridine, 2,2'-biquinoline, 2,2'-bi-4-lepidine, 2,9-dibutyl-1, 10-phenathroline, 3,4,7,8-tetramethyl-1,10-phenanthroline and 2,9-dibutyl-1,10-phenathroline, and more suitably 2,2'-bipyridine, 1,10-phenanthroline, basophenanthroline, 5,5'-dimetyl-2,2'-bipyridine, and 5-phenyl-2,2'-bipyridine.

In addition, in Formula (2), when the rare earth metal atom Ln represents Eu, n represents preferably an inter of 1 to 2, and more preferably an interger of 1.

The rare earth metal complex of the present invention can be prepared by an usual method. For example, the rare earth metal complex of the present invention can be easily obtained by reacting a rare earth metal compound with a β-diketone compound in the presence of a base.

The rare earth metal compound used to manufacture the rare earth metal complex is not particularly limited. Examples of the rare earth metal compound include inorganic compounds of rare earth metals, such as oxides, hydroxides, sulfides, fluorides, chlorides, bromides, iodides, sulfates, sulfites, disulfates, hydrogen sulfates, thiosulfates, nitrates, nitrites, phosphates, phosphites, hydrogen phosphates, dihydrogen phosphates, diphosphates, polyphosphates, (hexa)fluorophosphates, carbonates, hydrogen carbonates, thiocarbonates, cyanides, thiocyanides, borates, (tetra)fluoroborates, cyanates, thiocyanates, isothyanates, azides, nitrides, borides, silicates, (hexa)fluorosilicates, isopolyacids, heteropolyacids, or other condensed polyacid salts, and organic compounds thereof, such as alcoholates, thiolates, amides, imides, carboxylates, sulfonates, phosphonates, phosphinates, amino acid salts, carbamates or xanthogenates.

The rare earth metal complex of the present invention has a maximum absorption at a wavelength of preferably 350 nm or more, more preferably from 350 to 400 nm, and still more preferably from 355 to 375 nm.
The maximum absorption wavelength of the rare earth metal complex of the present invention is a wavelength attributable to the β-diketone compound. When the β-diketone compound has been coordinated to the rare earth metal atom, the absorption wavelength of an anion of the β-diketone compound, namely, a β-diketonate, is observed. To shift toward longer absorption wavelength of the β-diketonate, it is desirable to extend a conjugated system.

The β-diketone compound has a maximum absorption at a wavelength of preferably 345 nm or more, more preferably from 350 to 400 nm, and still more preferably from 355 to 375 nm.

The maximum absorption wavelength of the rare earth metal complex of the present invention is measured in a solution prepared such that the absorbance is 1 or less in a rectangular quartz cell with an optical path length of 1 cm using a commercially available spectrophotometer (for examples, U-3310 manufactued by Hitachi High-Tech Fielding Corporation). A desirable solvent for the measurement is a solvent having high sample solubility and low absorption in UV range. Examples of such a solvent include tetrahydrofuran, dimethylformaldehyde, and the like. Additionally, sample concentration for the Measurement is appropriately selected according to the molar absorption coefficient of each sample and is preferably adjusted such that the absorbance is in a range of from 0.1 to 1.0. In the present invention, the maximum absorption wavelength represents a value measured at a concentration of 2 × 10⁻⁵ [M] using dimethylformaldehyde as the solvent.

Additionally, the rare earth metal complex of the present invention has a maximum excitation at a wavelength of preferably from 395 to 450 nm, more preferably from 400 to 440 nm, and still more preferably from 405 to 435 nm.

The maximum excitation wavelength of the rare earth metal complex of the present invention is measured by fixing the wavelength of a fluorescence side spectroscope (particularly when the light emission center is made of Eu³⁺, the wavelength is appropriately adjusted in a range of from 605 to 620 nm representing a maximum light emission intensity) and scanning the wavelength of an excitation side spectroscope, using a commercially available spectrofluorophotometer (for example, F-4500, manufactured by Hitachi High-Technologies Corporatoin). The shape of the samples is selected from powder, solution, a state of having been dispersed in resin, and the like. The sample shape is not limited to any form in a relative comparison. Additionally, careful attention is required since samples in powder state scatter and samples in solution or dispersed in resin are affected by a medium or show dependency on the concentration of the medium. The maximum excitation wavelength in the present invention represents a value measured at a concentration of 1 × 10⁻⁴ [M] using dimethylformamide as the solvent.

The use of the rare earth metal complex of the present invention is not particularly limited. Examples of the use thereof include light-emitting probes, bioimaging, ink for printing, sensors, wavelength-converting resin sheet, lighting, and the like.
In addition, the rare earth metal complex of the present invention may be used, for example, as a resin sealing spherical fluorescent material by dispersing in resin or dissolving in a vinyl monomer for suspension polymerization, as well as may be applied to a wavelength-converting resin composition used on the light receiving surface side of a solar cell, a wavelength conversion type solar cell sealing material (wavelength conversion type solar cell sealing sheet), and solar cell modules using these components. For example, by using the rare earth metal complex of the present invention for these applications, light of a wavelength range less contributed to electric power generation is wavelength-converted to light of a wavelength range more contributed to electrical power generation, thereby improving power generation efficiency.

### [Examples]

Given hereinbelow is a more detailed description of the present invention with reference to Examples. The invention, however, is not limited thereto.

### [Example 1]

### < Synthesis of FTP [1-(4-fluorophenyl)-3-(2-thienyl)-1,3-propariedione] >

An amount of 0.96 g (0.04 mol) of sodium hydride was weighed out, and under a nitrogen atmosphere, 22.5 ml of dehydrated tetrahydrofuran was added. While strongly stirring the mixture, a solution of 2.52 g (0.02 mol) of 2-acetylthiophene and 3.70 g (0.024 mol) of methyl 4-fluorobenzoate dissolved in 12.5 ml of dehydrated tetrahydrofuran was added dropwise in 1 hour. Subsequently, the resulting mixture was subjected to reflux for 8 hours under a nitrogen gas flow. The reaction solution was returned to room temperature, 10.0 g of pure water was added, and furthermore, 5.0 ml of 3 mol/L hydrochloric acid was added. The organic layer was separated and concentrated under reduced pressure. The concentrate was recrystallized to obtain 2.83 g (a yield of 57%) of FTP as a β-diketone compound.

### < Synthesis of Eu(FTP)₃Phen >

In 25.0 g of methanol were dispersed 556.1 mg (2.24 mmol) of FTP synthesized as described above and 151.4 mg (0.84 mmol) of 1,10-phenanthroline (Phen). Into the dispersion was added a solution of 112.0 mg (2.80 mmol) of sodium hydroxide dissolved in 10.0 g of methanol, and the mixture was stirred for 1 hour.
Next, a solution of 256.5 mg (0.7 mmol) of europium (III) chloride hexahydrate dissolved in 5.0 g of methanol was added dropwise into the mixture. After stirring the resulting mixture at room temperature for 2 hours, a produced precipitate was suction-filtrated, washed with methanol, and then dried to obtain 730.0 mg of Eu(FTP)₃Phen.

### [Example 2]

### < Synthesis of TFTP [1-(4-(trifluoromethyl)phenyl)-3-(2-thienyl)-1,3-propanedione] >

An amount of 0.48 g (0.02 mol) of sodium hydride was weighed out, and under a nitrogen atmosphere, 20.0 ml of dehydrated tetrahydrofuran was added. While strongly stirring the mixture, a solution of 1.26 g (0.01 mol) of 2-acetylthiophene and 2.45 g (0.012 mol) of methyl 4-(trifluoromethyl)benzoate dissolved in 25.0 ml of dehydrated tetrahydrofuran was added dropwise in 1 hour. Subsquently, the resulting mixture was subjected to reflux for 8 hours under a nitrogen gas flow. The reaction solution was returned to room temperature, 10.0 g of pure water was added, and furthermore, 6.0 ml of 3 mol/L hydrochloric acid was added. The organic layer was separated and concentrated under reduced pressure. The concentrate was recrystallized to obtain 1.75 g (a yield of 59%) of TFTP as a β-diketone compound.

### <Synthesis of Eu(TFTP)₃Phen >

In 14.1 g of methanol were dispersed 377.1 mg (1.26 mmol) of TFTP synthesized as described above and 85.4 mg (0.47 mmol) of 1,10-phenanthroline (Phen). Into the dispersion was added a solution of 63.2 mg (1.58 mmol) of sodium hydroxide dissolved in 5.64 g of methanol, and the mixture was stirred for 1 hour.
Next, a solution of 144.8 mg (0.40 mmol) of europium (III) chloride hexahydrate dissolved in 2.82 g of methanol was added dropwise into the mixture. After stirring the resulting mixture at room temperature for 2 hours, a produced precipitate was suction-filtrated, washed with methanol, and then dried to obtain 458.0 mg of Eu(TFTP)₃Phen.

### [Example 3]

### < Synthesis of PTP [1-phenyl-3-(2-thienyl)-1,3-propanedione] >

An amount of 1.92 g (0.08 mol) of sodium hydride was weighed out, and under a nitrogen atmosphere, 45.0 ml of dehydrated tetrahydrofuran was added. While strongly stirring the mixture, a solution of 4.81 g (0.04 mol) of acetophenone and 7.50 g (0.048 mol) of 2-thiophene carboxylic acid ethyl dissolved in 50.0 ml of dehydrated tetrahydrofuran was added dropwise in 1 hour. Subsequently, the resulting mixture was subjected to reflux for 8 hours under a nitrogen gas flow. The reaction solution was returned to room temperature, 20.0 g of pure water was added, and furthermore, 16.0 ml of 3 mol/L hydrochloric acid was added. The organic layer was separated and concentrated under reduced pressure. The concentrate was recrystallized to obtain 4.79 g (a yield of 52%) of PTP as a β-diketone compound.

### < Synthesis of Eu(PTP)₃Phen >

In 25.0 g of methanol were dispersed 515.9 mg (2.24 mmol) of PTP synthesized as described above and 151.4 mg (0.84 mmol) of 1,10-phenanthroline (Phen). Into the dispersion was added a solution of 112.0 mg (2.80 mmol) of sodium hydroxide dissolved in 10.0 g of methanol, and the mixture was stirred for 1 hour.
Next, a solution of 256.5 mg (0.7 mmol) of europium (III) chloride hexahydrate dissolved in 5.0 g of methanol was added dropwise into the mixture. After stirring the resulting mixture at room temperature for 2 hours, a produced precipitate was suction-filtrated, washed with methanol, and then dried to obtain 645.0 mg of Eu(PTP)₃Phen.

### [Example 4]

### < Synthesis of Eu(PTP)₃Bpy >

In 25.0 g of methanol were dispersed 515.9 mg (2.24 mmol) of PTP synthesized as described above and 131.2 mg (0.84 mmol) of 2,2'-bipyridine (Bpy). Into the dispersion was added a solution of 112.0 mg (2.80 mmol) of sodium hydroxide dissolved in 10.0 g of methanol, and the mixture was stirred for 1 hour.
Next, a solution of 256.5 mg (0.7 mmol) of europium (III) chloride hexahydrate dissolved in 5.0 g of methanol was added dropwise into the mixture. After stirring the resulting mixture at room temperature for 2 hours, a produced precipitate was suction-Filtrated, washed with methanol, and then dried to obtain 563.4 mg of Eu(PTP)₃Bpy.

### [Example 5]

### < Synthesis of MTP [1-(4-methoxyphenyl)-3-(2-thienyl)-1,3-propanedione] >

An amount of 0.96 g (0.04 mol) of sodium hydride was weighed out, and under a nitrogen atmosphere, 22.5 ml of dehydrated tetrahydrofuran was added. While strongly stirring the mixture, a solution of 3.00 g (0.02 mol) of 4-methoxyacetophenone and 3.75 g (0.024 mol) of 2-thiophene carboxylic acid ethyl dissolved in 25.0 ml of dehydrated tetrahydrofuran was added dropwise in 1 hour. Subsequently, the resulting mixture was subjected to reflux for 8 hours under a nitrogen gas flow. The reaction solution was returned to room temperature, 10.0 g of pure water was added, and furthermore, 7.5 ml of 3 mol/L hydrochloric acid was added. The organic layer was separated and concentrated under reduced pressure. The concentrate was recrystallized to obtain 2.78 g (a yield of 53%) of MTP as a β-diketone compound.

### < Synthesis of Eu(MTP)₃Phen >

In 25.0 g of methanol were dispersed 583.1 mg (2.24 mmol) of MTP synthesized as described above and 151.4 mg (0.84 mmol) of 1,10-phenanthroline (Phen). Into the dispersion was added a solution of 112.0 mg (2.80 mmol) of sodium hydroxide dissolved in 10.0 g of methanol, and the mixture was stirred for 1 hour.
Next, a solution of 256.5 mg (0.7 mmol) of europium (III) chloride hexahydrate dissolved in 5.0 g of methanol was added dropwise into the mixture. After stirring the resulting mixture at room temperature for 2 hours, a produced precipitate was suction-filtrated, washed with methanol, and then dried to obtain 754.1 mg of Eu(MTP)₃Phen.

### [Example 6]

### < Synthesis of Eu(MTP)₃Bpy >

In 25.0 g of methanol were dispersed 583.1 mg (2.24 mmol) of MTP synthesized as described above and 131.2 mg (0.84 mmol) of 2,2'-bipyridine (Bpy). Into the dispersion was added a solution of 112.0 mg (2.80 mmol) of sodium hydroxide dissolved in 10.0 g of methanol, and the mixture was stirred for 1 hour.
Next, a solution of 256.5 mg (0.7 mmol) of europium (III) chloride hexahydrate dissolved in 5.0 g of methanol was added dropwise into the mixture. After stirring the resulting mixture at room temperature for 2 hours, a produced precipitate was suction-filtrated, washed with methanol, and then dried to obtain 710.4 mg of Eu(MTP)₃Bpy.

### [Comparative Example 1]

### < Synthesis of Eu(TTA)₃Phen >

In 11 g of sodium hydroxide (1M) was added a solution of 2.00 g (9.00 mmol) of thenoyltrifluoroacetone (TTA) dissolved in 75.0 g of ethanol. Next, a solution of 0.62 g (3.44 mmol) of 1,10-phenathroline dissolved in 75.0 g of ethanol was added, and the mixture was stirred continuously for 1 hour.
Next, a solution of 1.03 g (2.81 mmol) of europium (III) chloride hexahydrate dissolved in 20.0 g of ethanol was added dropwise to the mixtuer, and the resulting mixture was stirred continously for 1 more hour. A produced precipitate was suction-filtrated, washed with ethanol, and dried to obtain 2.33 g of Eu(TTA)₃Phen.

### [Comparative Example 2]

### < Synthesis of Eu(BFA)₃Phen >

In 11g of sodium hydroxide (1M) was added a solution of 1.94 g (9.00 mmol) of benzoyltrifluoroacetone (BFA) dissolved in 60.0 g of ethanol. Next, a solution of 0.62 g (3.44 mmol) of 1,10-phenathroline dissolved in 60.0 g of ethanol was added, and the mixture was stirred continuously for 1 hour.
Next, a solution of 1.03 g (2.81 mmol) of europium (III) chloride hexahydrate dissolved in 20.0 g of ethanol was added dropwise to the mixture, and the resulting mixture was stirred continously for 1 more hour. A produced precipitate was suction-filtrated, washed with ethanol, and then dried to obtain 2.22 g of Eu(BFA)₃Phen.

### [Measurement Methods]

The following is a description of methods for measuring individual parameters, such as excitation wavelengths measured regarding the rare earth metal complexes obtained above.

### 1. Measurement of Maximum Absorption Wavelength

Using the spectrophotometer, U-3310, manufactured by Hitachi High-Tech Fielding Corporation, the maximum absorption wavelength was measured at the concentration of 2 × 10⁻⁵ [M] using dimethylformaldehyde as the solvent.
FIG.. 1 illustrates the maximum absorption wavelengths of the rare earth metal complexes obtained in Example 1, and Comparative Examples 1 and 2.

### 2. Measurement of Maxiumum Excitation Wavelength

Using the spectrofluorophotometer, F-4500, manufactured by Hitachi High-Technologies Corporatoin, the maximum excitation wavelength was measured at the concentration of 2 × 10⁻⁴ [M] using dimethylformaldehyde as the solvent.
FIG.. 2 illustrates the excitation spectra of the rare earth metal complexes obtained in Example 1, and Comparative Examples 1 and 2.

**Table 1**

| | β-diketone compound | Maximum Absorption Wavelength (nm) | Eu complex | Maximum Absorption Wavelength (nm) | Maximum Excitation Wavelength (nm) |
|---|---|---|---|---|---|
| Example 1 | FTP | 359 | Eu(FTP)₃Phen | 363 | 425 |
| Example 2 | TFTP | 360 | Eu(TFTP)₃Phen | 370 | 434 |
| Example 3 | PTP | 360 | Eu(PTP)₃Phen | 365 | 428 |
| Example 4 | PTP | 360 | Eu(PTP)₃Bpy | 365 | 429 |
| Example 5 | MTP | 373 | Eu(MTP)₃Phen | 368 | 429 |
| Example 6 | MTP | 373 | Eu(MTP)₃Bpy | 368 | 428 |
| Comparative Example 1 | TTA | 344 | Eu(TTA)₃Phen | 341 | 391 |
| Comparative Example 2 | BFA | 331 | Eu(BFA)₃Phen | 325 | 375 |

As shown in Table 1, it is apparent that the rare earth metal complexes of Examples 1 to 6 including the β-diketone compound represented by Formula (1) as the ligand have been excited by excitation light having longer wavelengths than in the rare earth metal complexes of Comparative Examples 1 and 2 that do not include the β-diketone compound represented by Formula (1) as the ligand.

The disclosure of Japanese Application No. 2010-260326 is incorporated herein by reference in its entirety.
All literatures, patent applications and technical standards described in the present specification are herein incorporated by reference to the same extent as if each individual literature, patent application and technical standard was specifically and individually indicated as being incorporated by reference.

## Claims

1. A rare earth metal complex comprising:
a rare earth metal atom; and
a β-diketone compound coordinated to the rare earth metal atom, the β-diketone compound being represented by the following Formula (1): 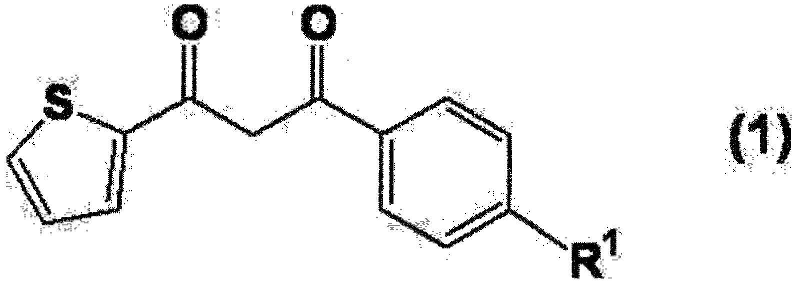 wherein, in Formula (1), R¹ represents a hydrogen atom, a halogen atom, an alkyl group, a perfluoroalkyl group, an alkoxy group, a perfluoroalkoxy group, a nitro group, an amino group, a sulfonyl group, a cyano group, a silyl group, a phosphone group, a diazo group, a mercapto group, an aryl group, an aralkyl group, an aryloxy group, an aryloxycarbonyl group, an allyl group, an acyl group or an acyloxy group.

2. The rare earth metal complex according to claim 1, having maximum absorption at a wavelength of 350 nm or more.

3. The rare earth metal complex according to claim 1 or 2, represented by the following Formula (2): 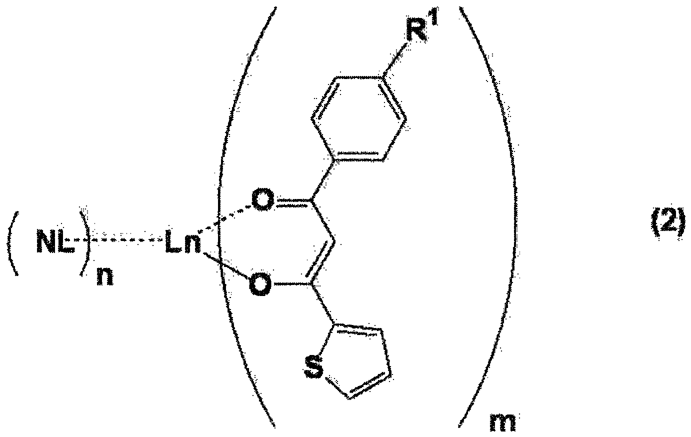 wherein, in Formula (2), Ln represents the rare earth metal atom; NL represents a neutral ligand; R¹ represents a hydrogen atom, a halogen atom, an alkyl group, a perfluoroalkyl group, an alkoxy group, a perfluoroalkoxy group, a nitro group, an amino group, a sulfonyl group, a cyano group, a silyl group, a phosphone group, a diazo group, a mercapto group, an aryl group, an aralkyl group, an aryloxy group, an aryloxycarbonyl group, an allyl group, an acyl group, or an acyloxy group; n represents an integer from 1 to 5; and m represents an integer equal to a valence of Ln.

4. The rare earth metal complex according to any one of claims 1 to 3, wherein the rare earth metal atom is europium (Eu), terbium (Tb), erbium (Er), ytterbium (Yb), neodymium (Nd) or samarium (Sm).

5. The rare earth metal complex according to any one of claims 1 to 4, wherein R¹ in Formula (1) represents an electron attracting group.

6. The rare earth metal complex according to any one of claims 1 to 4, wherein R¹ in Formula (1) represents a halogen atom or a perfluoroalkyl group.
